# EUROPEAN PATENT APPLICATION

(11) **EP 2 108 643 A1**
(43) Date of publication of application: **14.10.2009**
(21) Application number: 08384004.1
(22) Date of filing: 10.04.2008
(51) Int. Cl.: C07D 231/06, A61K 31/44, A61P 3/04

(54) **4-methyl-4, 5-dihydro-1H-pyrazole-3-carboxamide useful as a cannabinoid CB1 neutral antagonist**

(71) Applicant: Laboratorios Del. Dr. Esteve, S.A., 08041 Barcelona (ES)
(72) Inventor: Vela Hernandez, Jose Miguel, 08028 Barcelona (ES); Yenes Minguez, Susana, 08750 Molins de Rei (ES)
(74) Representative: Ponti Sales, Adelaida

(57) **Abstract**

The present invention relates to 4-methyl-4,5-dihydro-1H-pyrazole-3-carboxamide, and pharmaceutically acceptable salts and solvates thereof. It further concerns pharmaceutical compositions comprising this compound as active ingredient as well as processes and intermediates for preparing this compound and compositions. The referred compound is a cannabinoid CB₁ neutral antagonist useful in the prophylaxis and treatment of food intake disorders.

## Description

### Field of the invention

The present invention relates to 4-methyl-4,5-dihydro-1H-pyrazole-3-carboxamide, and pharmaceutically acceptable salts and solvates thereof. It further concerns pharmaceutical compositions comprising this compound as active ingredient as well as processes and intermediates for preparing this compound and compositions. The referred compound is a cannabinoid CB₁ neutral antagonist useful in the prophylaxis and treatment of food intake disorders.

### Background of the invention

Diseases characterised by impaired energy balance, such as obesity, are among the leading causes of illness and mortality in developed countries. Understanding the complex network of central and peripheral factors that influence both appetite and energy expenditure is a major public health goal. There is increased evidence suggesting that the cannabinoid system is involved in the regulation of food intake and appetite (Cani et al. 2004).

Mammalian tissues express at least two types of cannabinoid receptors, CB₁ and CB₂, both G protein coupled (reviewed in Howlett et al., 2002). CB₁ receptors, aka CNR1, are found predominantly at central and peripheral nerve terminals and mediate inhibition of transmitter release whereas CB₂ receptors are found mainly on immune cells, one of their roles being to modulate cytokine release. Endogenous ligands -or "endocannabinoids"- for these receptors also exist (Pertwee 2004). These "endocannabinoids" together with their receptors and the biosynthetic and degradative enzymes for these ligands constitute the "endocannabinoid system", which is involved in feeding and metabolism. These pathways include vagal afferents, area postrema and brain stem nuclei, hypothalamus, and reward pathways. In the periphery, CB₁ receptors and their ligands are also found in the gut, liver, and adipose tissue. Vagal afferents, area postrema, and brain stem are also sites involved in emesis (Chambers et al., 2007).

The discovery of this system of receptors and endogenous messengers prompted the development of CB₁- and CB₂-selective agonists, mixed CB₁/CB₂ receptor agonists, such as CP55940, R-(+)-WIN55212 and Δ⁹-tetrahydrocannabinol (Δ⁹-THC) (reviewed in Howlett et al., 2002), and selective antagonists the first of which were the CB₁-selective SR141716A, AM251, AM281 and LY320135, and the CB₂-selective SR144528 and AM630 (reviewed in Howlett et al., 2002). SR141716A, AM251, AM281, and LY320135 all behave as inverse agonists, producing effects in some CB₁ containing bioassay systems that are opposite in direction from those produced by agonists for these receptors.

The stimulation of appetite by cannabinoid agonists has been widely reported, both in marijuana consumers and in cachectic patients. In contrast, CB₁ cannabinoid receptor antagonists have been shown to reduce appetite and body weight in animal models and in human subjects (Cani et al. 2004).

According to the International Union of Pharmacology Committee on Receptor Nomenclature and Drug Classification, an agonist is a ligand that binds to a receptor and alters the receptor state resulting in a biological response. Conventional agonists increase receptor activity, whereas inverse agonists reduce it. As such, an inverse agonist is defined as a ligand that by binding to receptors reduces the fraction of them in an active conformation. Inverse agonists show a preferential binding affinity for the inactive conformation of the receptor. In systems where constitutive activity of the receptor can be seen, inverse agonists induce the opposite action of that of agonists.

The mentioned article above defines an antagonist as a drug that reduces the action of another drug, generally an agonist. Many antagonists act at the same receptor macromolecule as the agonist. In turn, a neutral antagonist refers to a ligand that binds to the receptor and is able to counteract the actions of agonists and inverse agonists for the same receptor impairing their responses. The neutral antagonist is not able to induce any activity by its own as it cannot discriminate between the different active and inactive conformations of the receptor.

In practice, the difference between an inverse agonist and a neutral antagonists lies on the action they effect towards the basal receptor activity. While the inverse agonist decreases this basal receptor activity, i.e. Eₘᵢₙ is negative, the neutral antagonist does not, it simply does not affect said basal receptor activity, i.e. Eₘᵢₙ is approximately 0. Eₘᵢₙ refers to the minimum effect or action that a certain ligand can produce.

CB₁ inverse agonists present a negative Eₘᵢₙ value, corresponding to the decrease of endogenous basal receptor activity, and is associated with undesired side effects including depressive disorders, anxiety, mood alterations with depressive symptoms, nausea, and dizziness. Like the CB₁ inverse agonists, CB₁ neutral antagonists decrease appetite but without inducing the side-effects associated with inverse agonists (Sink et al., 2008).

Inverse agonists and neutral antagonists of the CB₁ receptor may act on the central nervous system (CNS), on the peripherical system, or both. Those ones which can cross the Blood Brain Barrier (BBB) will act on both the CNS and the peripherical system. Crossing the BBB and acting on the CNS presents undesirable effects such as depressive disorders, anxiety, mood alterations with depressive symptoms, nausea, and dizziness. Those ligands which do not cross the BBB will act only on the peripherical system, which by means of gut hormones or nerve afferents will indirectly interact with the CNS to reduce appetite but without inducing the undesired side effects derived from the direct action at the CNS.

Ideally, a candidate compound for reducing appetite would be a CB₁ neutral antagonist that does not cross the BBB.

WO07/009689 relates to 4-substituted pyrazoline compounds, methods for their preparation, medicaments comprising these compounds as well as their use for the preparation of a medicament for the treatment of humans and animals. The medicament is suitable for the prophylaxis and/or treatment of food intake disorders, such as bulimia, anorexia, cachexia, obesity, type II diabetes mellitus, and appetency disorders. It particularly lists the racemate compound denominated cis-5-(4-chlorophenyl)-1-(2,4-dichlorophenyl)-N-(2,6-dimethylpiperidin-1-yl)-4-methyl-4,5-dihydro-1H-pyrazole-3-carboxamide (see compound nr 103 on page 229). It is relevant to note that such denomination embraces 4 isomers resulting from the chiral carbon atoms 4 and 5 of the pyrazoline ring, and from the carbon atoms 2 and 6 of the piperidine ring.

While the racemate compound mentioned above and its enantiomer (4R,5R) show CB₁ inverse agonism, it has been surprisingly found that the specific isomers embraced by (4S,5S)-5-(4-chlorophenyl)-1-(2,4-dichlorophenyl)-N-(2,6-dimethylpiperidin-1-yl)-4-methyl-4,5-dihydro-1H-pyrazole-3-carboxamide act as CB₁ neutral antagonists. More surprisingly, these specific isomers (4S,5S) do not cross the BBB, acting exclusively on the peripherical system, avoiding thereby the undesired side effects.

### Summary of the invention

The present invention relates to a compound having the formula (I) 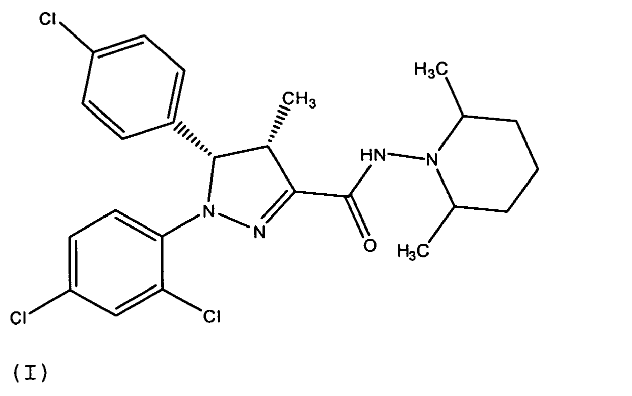 and pharmaceutically acceptable salts and solvates thereof.

In one embodiment, compound of formula (I) is (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide.

The invention further relates to a process for the preparation of the compounds of the invention as well as to intermediates useful in the preparation of the mentioned compound.

The invention further provides a pharmaceutical composition comprising a compound of the invention and a pharmaceutically acceptable carrier. Said compounds may be used as a medicament, in particular for decreasing body weight.

### Description of the figures

Figure 1 is a graph representing the percentage of inhibition of the [³⁵S]GTPγS binding to the CB₁ receptor in the presence of Rosonabant, a known inverse agonist described in WO2005077911; in the presence of cis-5-(4-chlorophenyl)-1-(2,4-dichlorophenyl)-N-(2,6-dimethylpiperidin-1-yl)-4-methyl-4,5-dihydro-1H-pyrazole-3-carboxamide (denominated as the racemate cis-compound); and in the presence of (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, denominated as the (4S,5S) compound.
Figure 2 is a Chiral HPLC chromatogram for enantiomer (4*S*,5*S*)-5-(4-chlorophenyl)-1-(2,4-dichlorophenyl)-4-methyl-4,5-dihydro-1H-pyrazole-3-carboxylic acid after the resolution of the racemate.
Figure 3 is a Chiral HPLC chromatogram for the other enantiomer (4*R*,5*R*)-5-(4-chlorophenyl)-1-(2,4-dichlorophenyl)-4-methyl-4,5-dihydro-1H-pyrazole-3-carboxylic acid after the resolution of the racemate.

### Disclosure of the invention

The present invention relates to a compound having the formula (I) 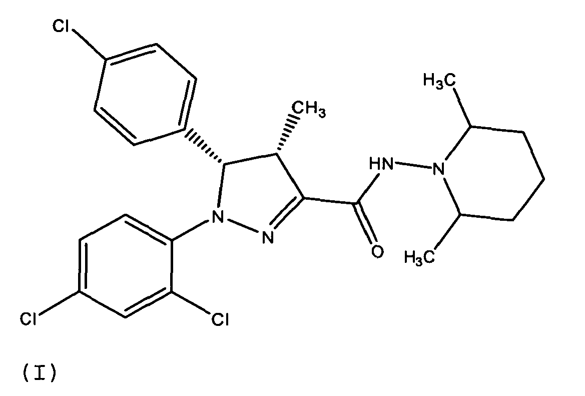 and pharmaceutically acceptable salts and solvates thereof.

Said compound is also named (4S,5S)-5-(4-chlorophenyl)-1-(2,4-dichlorophenyl)-N-(2,6-dimethylpiperidin-1-yl)-4-methyl-4,5-dihydro-1H-pyrazole-3-carboxamide.

The compound of the invention and intermediates thereof present two asymmetric centers at positions 4 and 5 of the pyrazoline ring. As indicated on the formulae depicted herein and the chemical name, these asymmetric centers occur each in the S configuration.

The compound of the invention and intermediates thereof present as well an alicyclic group, i.e. the 2,6-dimethylpiperidine, which displays a cis-trans isomerism due to the presence of 2 methyl substituents. As such, the 2,6-dimethylpiperidine may occur in the trans or cis configuration.

The two possible isomers resulting from the cis-trans configuration are the object of the present invention. As such, in one embodiment, the present invention relates to the compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide. In another embodiment, the present invention relates to the compound (4S,5S)-5-(4-chlorophenyl)-1-(2,4-dichlorophenyl)-N-(trans-2,6-dimethylpiperidin-1-yl)-4-methyl-4,5-dihydro-1H-pyrazole-3-carboxamide.

The pure stereoisomeric form of the compound of the invention may be obtained by the application of art-known procedures. For instance, enantiomers may be separated from each other by treating the corresponding racemic isomers mixture by usual optical resolution methods such as fractional recrystallization method, optical resolution column chromatography method such as liquid chromatography using a chiral stationary phase, or diastereomer method; or separation methods such as recrystallization method, column chromatography method, amongst other. For example, when optical isomers are to be separated, the racemic mixture of the compound the invention may be reacted with an optically active acid or base. Examples thereof are tartaric acid, dibenzoyl-tartaric acid, ditoluoyltartaric acid, and camphosulfonic acid. Said pure stereochemically isomeric form of the compound of the present invention may also be derived from the corresponding pure stereochemically isomeric form of the appropriate starting materials or intermediates, provided that the reaction occurs stereospecifically. Also, when the above reaction is carried out by using the starting material or intermediate that has been subjected to optical resolution or separation, the desired optical isomer or geometric isomer can be obtained.

The term salt as mentioned herein is meant to comprise any stable salts, which the compound above is able to form. The salts can conveniently be obtained by treating the base form with such appropriate acids as inorganic acids, for example, hydrohalic acids, e.g. hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propane-tricarboxylic, methanesulfonic, ethanesulfonic, benzenesulfonic, 4-methylbenzene-sulfonic, cyclohexanesulfamic, 2-hydroxybenzoic, 4-amino-2-hydroxybenzoic, and the like acids. Conversely the salt form can be converted by treatment with alkali into the free base form.

The compound of the present invention may also be converted into its non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term salts is also meant to include the hydrates or solvates which the compound of the present invention is able to form, including, e.g. the alcoholates such as methanolates or ethanolates.

The phrase "pharmaceutically acceptable" refers to molecular entities and compositions that do not produce adverse, allergic, or other untoward reactions when administered to an animal or a human.

In one embodiment of the present invention, the compound of the present invention is in a salt form, preferably the salt is chloride or hydrochloride.

The term "solvate" refers to those crystal forms of the compound of the present invention that contain either stoichiometric or non-stoichiometric amounts of solvent. Since water is a solvent, solvates also include hydrates. The term "pseudopolymorph" is synonym to solvate since it applies to polymorphic crystalline forms that have solvent molecules incorporated in their lattice structures. Examples of solvates are hydrates and alcoholates such as methanolates or ethanolates.

In another embodiment, the present invention provides a process for the preparation of a compound of formula (I), said process comprising the step of:
a1) coupling a compound of formula (II) with compound of formula (IV) in a suitable solvent, in the presence of a coupling agent, and optionally in the presence of an activating agent of the coupling agent, 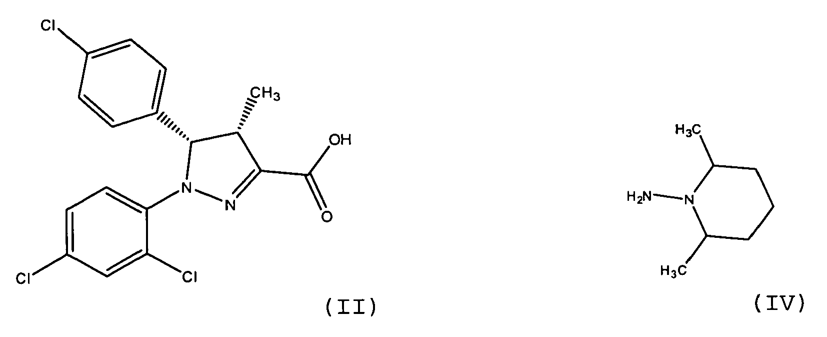 or
a2) converting a compound of formula (II) into a compound of formula (III) with an activating agent, and coupling said compound of formula (III) with compound of formula (IV) in a suitable solvent, 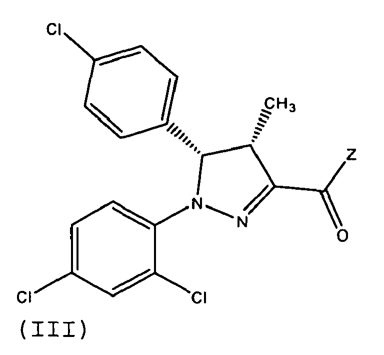 wherein
   **Z** represents halo, -O-CO-R¹, or -O-CO-OR¹; and
   **R¹** is C₁₋₄alkyl, aryl, or benzyl;
   thereby obtaining compound of formula (I).

The reaction can be carried out using the *cis* or *trans* configuration of the compound of formula (IV) to obtain the (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, or the (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide, respectively.

As such in one embodiment, the present invention provides a process for the preparation of (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, said process comprising the step of:
a1) coupling a compound of formula (II) with *cis*-2,6-dimethylaminopiperidine in a suitable solvent, in the presence of a coupling agent, and optionally in the presence of an activating agent of the coupling agent, 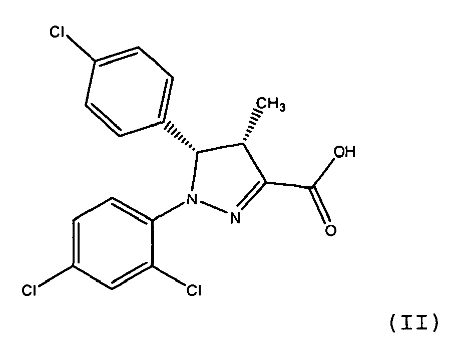 or
a2) converting a compound of formula (II) into a compound of formula (III) with an activating agent, and coupling said compound of formula (III) with *cis*-2,6-dimethylaminopiperidine in a suitable solvent, 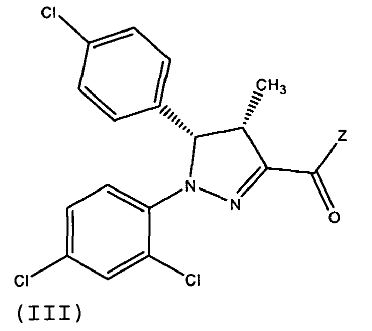 wherein
   **Z** represents halo, -O-CO-R¹, or -O-CO-OR¹; and
   **R¹** is C₁₋₄alkyl, aryl, or benzyl;
   thereby obtaining compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide.

The term halo is generic to fluoro, chloro, bromo, and iodo.

As used hereinbefore or hereinafter C₁₋₄alkyl as a group or part of a group defines straight or branched chain saturated hydrocarbon radicals having from 1 to 4 carbon formula (III), with the use of an activating agent according to the methods known by the skilled in the art. Such activating agent may be selected from a halogenating agent, C₁₋₄alkyl acid halide, aryl acid halide, benzyl acid halide, C₁₋₄alkyl haloformate, aryl haloformate, and benzyl haloformate.

Examples of halogenating agents that may be used include, but are not limited to, inorganic acid halides, thionyl chloride, cyanuric chloride, Vilsmeier reagent, Gold's reagent, chlorinated heterocycles, and combinations of halogenating agents such as halogens, CCl₄, C₂Cl₆, or other alkyl halides with reducing agents such as triaryl or trialkyl phosphines or phosphites or a hydrogen halide in the presence of a dehydrating agent. Examples of C₁₋₄alkyl acid halides include acetyl chloride, propionyl chloride, butanoyl chloride, and the like. Examples of aryl acid halides include phthaloyl chloride, isophthaloyl chloride, and terphthaloyl chloride. Examples of benzyl acid halides include benzoyl chloride, benzoyl fluoride, benzoyl bromide. Examples of C₁₋₄alkyl haloformate include methyl chloroformate, ethyl chloroformate, isopropyl chloro-formate, and the like. Examples of aryl haloformate include phenyl chloroformate, phenyl fluoroformate, and the like. Examples of benzyl haloformates include benzyl chloroformate, benzyl fluoroformate, and the like.

In one embodiment of the present invention, when compound of formula (II) is activated as a compound of formula (III), in which Z is halo, it may be convenient to add a base to the reaction mixture in order to neutralize a possible *in situ* formed acid, such as hydrochloric acid.

In many instances, the coupling reaction of step a1) can be enhanced by the addition of an activating agent of the coupling agent that can be selected from 1-hydroxybenzotriazole (HOBt) and 4-dimethylaminopyridine (4-DMAP).

The coupling reaction of step a1) can optionally be performed in the presence of a base, preferably in the presence of a base selected from the group consisting of pyridine, dimethylaminopyridine, N-methylmorpholine, triethylamine, and diisopropylethylamine (DIPEA).

Preferably said reaction is carried out in the presence of EDCI and HOBt, optionally in the presence of N-methylmorpholine or triethylamine, in an aprotic reaction medium such as dimethylformamide or tetrahydrofuran, at a temperature between 20 °C and 30 °C for 15 to 24 hours as described in Tetrahedron Left. 2004, 45, 4977. The respective description is hereby incorporated by reference and forms part of the disclosure. Polymer-supported EDCI (P-EDCI) can also suitably be used for this process instead of EDCI as described in Tetrahedron Left. 1998, 39, 1487 and Tetrahedron Left. 2002, 43, 7685. The respective descriptions are hereby incorporated by reference and form part of the disclosure.

Alternatively said reaction can be carried out by using HBTU in the presence of a base such as diisopropylethylamine in an aprotic solvent, such as acetonitrile, preferably at a temperature between 20° and 30 °C for 15 to 24 hours.

According to step a2) of the process for the preparation of compound of formula (V), compound of formula (II) may be converted into an activated form, i.e. compound of atoms such as methyl, ethyl, propyl, 1-methylethyl, and butyl.

Aryl as a group or part of a group is phenyl or naphthyl.

The coupling reactions of each of steps a1) and a2) are conducted according to the methods known by the skilled in the art in a suitable solvent. Such suitable solvent is usually of inert nature, such as halogenated hydrocarbons, e.g. dichloromethane, chloroform, dipolar aprotic solvents such as acetonitrile, dimethylformamide, dimethylacemide, ethers such as tetrahydrofuran, diethyether, alcohols such as methanol, ethanol, chlorohydrocarbons such as 1,2-dichloroethane, dichloromethane, and the like.

The coupling reaction of step a1) is performed in the presence of a coupling agent according to the methods known by the skilled in the art. Such coupling agent may be selected from dicyclohexylcarbodiimide (DCC), N,N'-carbonyl-diimidazole (CDI), 2-ethoxy-1-ethoxycarbonyl-1,2-dihydroquinoline (EEDQ), 1-isobutyloxycarbonyl-2- isobutyloxy-1,2-dihydroquinoline (IIDQ), 1-(3-dimethylaminopropyl)-3-ethylcarbodiimide (EDCI), diisopropylcarbodiimide, N-ethyl-N'-[(3dimethylamino)propyl]carbodiimide (EDC), EDC hydrochlorate, 1-ethyl-3-(3-dimethylaminopropyl)carbodiimide hydrochloride (EDAC), benzotriazol-1-yl-oxy-tris-pyrrolidino-phosphonium hexafluorophosphate (BOP), 1,1'-carbonyl-diimidazole (CDI), N-[(dimethyamino)-IH-1, 2, 3-triazolo[4, 5-b]pyridino-1-ylmethylen]-N-methylmethanaminium hexafluorophosphate N-oxid (HATU), benzotriazol-1-yl-oxy-tris-(dimethyl-amino)-phosphonium hexafluorophosphate, 2-(1H-benzotriazol-1-yl)-N,N,N',N'-tetramethyluronium tetrafluoroborate (TBTU), O-(benzotriazol-1-yl)-N, N, N', N'-tetramethyluroniom hexafluorophosphate (HBTU), and 1-hydroxy-7-azabenzotriazol (HOAt).

In one embodiment of the present invention, compound of formula (II) is obtained by chiral HPLC separation or by diastereomeric resolution with chiral amines of racemate *cis*-5-(4-chlorophenyl)-1-(2,4-dichlorophenyl)-4-methyl-4,5-dihydro-1H-pyrazole-3-carboxylic acid.

Racemate *cis*-5-(4-chlorophenyl)-1-(2,4-dichlorophenyl)-4-methyl-4,5-dihydro-1H-pyrazole-3-carboxylic acid can be prepared as described in WO07/009689. It can be obtained from reacting 4-(4-chlorophenyl)-3-methyl-2-oxo-3-butenoic acid with 2,4-dichlorophenyl hydrazine or a salt thereof in acetic acid, isopropanol, and the like, and heating to allow cyclisation and formation of the pyrazoline ring.

*Cis*-2,6-dimethyl-1-piperidinamine (CAS registry nr 39135-39-2) is commercially available from 3B Scientific Corporation, Pfaltz & Bauer Chemicals, Aldrich, Amfinecom, amongst other.

A further embodiment of the present invention refers to a compound of formula (II), *per se,*

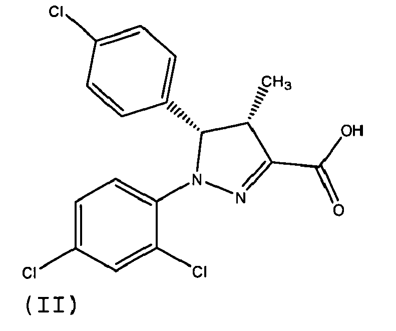

Another embodiment of the present invention refers to the use of compound of formula (II) as an intermediate in the preparation of a compound of formula (I); in the preparation of compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide; or in the preparation of compound (45,55)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide; pharmaceutically acceptable salts, prodrugs, solvates, and crystals thereof, as referred to herein above.

Another embodiment of the present invention refers to a compound of formula (III), *per se,* 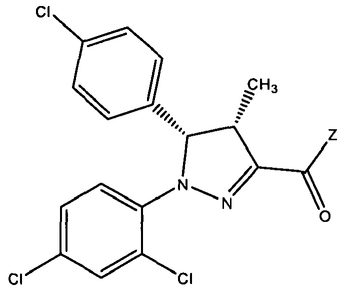 wherein
**Z** represents halo, -O-CO-R¹, or -O-CO-OR¹; and
**R¹** is C₁₋₄alkyl, aryl, or benzyl.

In particular, the invention refers to a compound of formula (IIIa), *per se,*

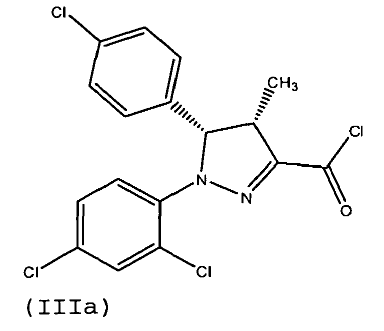

As such, compound of formula (III) and (IIIa) are useful as intermediates in the preparation of a compound of formula (I); in the preparation of compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide; or in the preparation of compound (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide; pharmaceutically acceptable salts, prodrugs, solvates, and crystals thereof, as referred to herein above.

In one embodiment of the present invention, the intermediate racemate *cis*-5-(4-chlorophenyl)-1-(2,4-dichlorophenyl)-4-methyl-4,5-dihydro-1*H*-pyrazol-3-carboxylic acid may be obtained in crystalline form, for instance by crystallization from toluene.

Intermediate racemate *cis*-5-(4-chlorophenyl)-1-(2,4-dichlorophenyl)-4-methyl-4,5-dihydro-1*H*-pyrazol-3-carboxylic acid exhibits an infrared (IR) spectrum comprising the following peaks: 2976, 1682, 1566, 1542, 1490, 1270, 1242, and 1117 cm⁻¹, with ± about 2 cm⁻¹ peak variation being allowed.

The term "crystalline" is defined as a form in which the position of the molecules relative to one another is organised according to a three-dimensional lattice structure.

Intermediate (4S, 5S)-5-(4-chlorophenyl)-1-(2,4-dichlorophenyl)-4-methyl-4,5-dihydro-1*H*-pyrazol-3-carboxylic acid can be obtained from racemate *cis*-5-(4-chlorophenyl)-1-(2,4-dichlorophenyl)-4-methyl-4,5-dihydro-1*H*-pyrazol-3-carboxylic acid by separation using chiral HPLC, in particular with a Chiralpak AD-H 5 µm column, or by Diastereomeric resolution with chiral amines, in particular with (+)-cinchonine.

Intermediate (4*S*,5*S*)-5-(4-chlorophenyl)-1-(2,4-dichlorophenyl)-4-methyl-4,5-dihydro-1H-pyrazole-3-carbonyl chloride may be obtained by reacting (4S, 5S)-5-(4-chlorophenyl)-1-(2,4-dichlorophenyl)-4-methyl-4,5-dihydro-1*H*-pyrazol-3-carboxylic acid with an activating agent, in particular with thionyl chloride.

Intermediate (4*S*,5*S*)-5-(4-chlorophenyl)-1-(2,4-dichlorophenyl)-4-methyl-4,5-dihydro-1H-pyrazole-3-carbonyl chloride exhibits an infrared (IR) spectrum comprising the following peaks: 1732, 1700, 1533, 1478, 1212, and 826 cm⁻¹, with ± about 2 cm⁻¹ peak variation being allowed.

The invention further provides a process for the preparation of intermediate racemate *cis*-5-(4-chlorophenyl)-1-(2,4-dichlorophenyl)-4-methyl-4,5-dihydro-1*H*-pyrazol-3-carboxylic acid, in crystalline form, said process comprising the steps of:
a) dissolving amorphous intermediate racemate *cis*-5-(4-chlorophenyl)-1-(2,4-dichlorophenyl)-4-methyl-4,5-dihydro-1*H*-pyrazol-3-carboxylic acid in an organic solvent, mixtures of organic solvents, water, or mixtures of water and water miscible organic solvents;
b) applying any suitable technique to induce crystallization; and
c) isolating the desired crystals.

By techniques for inducing crystallization are to be understood those processes for the production of crystals, which include amongst others, dissolving or dispersing the compound to be crystallized in a solvent medium, bringing the solution or dispersion of the compound and the solvent(s) to a desired concentration, bringing the said solution or dispersion to a desired temperature, effecting any suitable pressure, removing and/or separating any undesired material or impurities, drying the formed crystals to obtain the crystals in a solid state, if such state is desired.

Bringing the solution or dispersion of the compound and solvents to a desired concentration does not necessarily imply an increase in the concentration of the compound. In certain cases, a decrease or no change in concentration could be preferable. By bringing the said solution or dispersion to a desired temperature, one will understand the acts of heating, cooling, or leaving at ambient temperature.

The techniques used for obtaining a desired concentration are those common in the art, for instance, evaporation by atmospheric distillation, vacuum distillation, fractioned distillation, azeotropic distillation, film evaporation, other techniques well known in the art, or combinations thereof. An optional process for obtaining a desired concentration could as well involve the saturation of the solution of the compound to be crystallized and solvent, for example, by adding a sufficient volume of a nonsolvent to the solution to reach the saturation point. Other suitable techniques for saturating the solution include, by way of example, the introduction of additional compound to be crystallized to the solution and/or evaporation of a portion of the solvent from the solution. As referred to herein, saturated solution encompasses solutions at their saturation points or exceeding their saturation points, i.e. supersaturated.

Removing and/or separating any undesired material or impurities may be performed by purification, filtering, washing, precipitation, or similar techniques.

Separation, for example, can be conducted by known solid-liquid separation techniques. Filtering procedures known to those skilled in the art can as well be used in the present process. The filtrations can be performed, amongst other methods, by centrifugation, or using Buchner style filter, Rosenmund filter or plates, or frame press. Preferably, in-line filtration or safety filtration may be advantageously intercalated in the processes disclosed above, in order to increase the purity of the resulting crystal form. Additionally, filtering agents such as silica gel, Arbocel, dicalite diatomite, or the like, may also be employed to separate impurities from the crystals of interest.

Crystals obtained may be also dried, and such drying process may optionally be used in the different crystallization passages, if more than one crystallization passage is applied. Drying procedures include all techniques known to those skilled in the art, such as heating, applying vacuum, circulating air or gas, adding a desiccant, freeze-drying, spray-drying, evaporating, or the like, or any combination thereof.

In a further aspect, the present invention concerns a pharmaceutical composition comprising a therapeutically effective amount of a compound of formula (I), compound (4S,5S)-5-(4-chlorophenyl)-1-(2,4-dichlorophenyl)-N-(*cis-*2,6-dimethylpiperidin-1-yl)-4-methyl-4,5-dihydro-1H-pyrazole-3-carboxamide, or compound (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide, and a pharmaceutically acceptable carrier. A therapeutically effective amount in this context is an amount sufficient to prophylactically act against, to stabilize or to reduce body weight, in subjects being at risk of being infected. In still a further aspect, this invention relates to a process of preparing a pharmaceutical composition as specified herein, which comprises intimately mixing a pharmaceutically acceptable carrier with a therapeutically effective amount of a compound of formula (I), compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, or compound (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide.

Therefore, the compounds of the present invention may be formulated into various pharmaceutical forms for administration purposes. As appropriate compositions there may be cited all compositions usually employed for systemically administering drugs. To prepare the pharmaceutical compositions of this invention, an effective amount of the compounds of the present invention, optionally in a pharmaceutically acceptable salt or solvate thereof, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets.

Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

The compound of the present invention may also be administered via oral inhalation or insufflation by means of methods and formulations employed in the art for administration via this way. Thus, in general the compound of the present invention may be administered to the lungs in the form of a solution, a suspension or a dry powder, a solution being preferred. Any system developed for the delivery of solutions, suspensions or dry powders via oral inhalation or insufflation are suitable for the administration of the present compound.

Thus, the present invention also provides a pharmaceutical composition adapted for administration by inhalation or insufflation through the mouth comprising a compound of the present invention and a pharmaceutically acceptable carrier. The compounds of the present invention may be administered via inhalation of a solution in nebulized or aerosolized doses.

Depending on the mode of administration, the pharmaceutical composition will preferably comprise from 0.05 to 99 % by weight, more preferably from 0.1 to 70 % by weight, even more preferably from 0.1 to 50 % by weight of the active ingredient, and, from 1 to 99.95 % by weight, more preferably from 30 to 99.9 by weight %, even more preferably from 50 to 99.9 by weight % of a pharmaceutically acceptable carrier, all percentages being based on the total composition.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The daily dosage of the compounds according to the invention will, of course, vary with the mode of administration, the treatment desired and the severity of the food intake disorder. However, in general, satisfactory results will be obtained when the compounds according to the invention are administered at a daily dosage not exceeding 1 gram, e.g. in the range from 1 to 50 mg/kg body weight, preferably from 5 to 50 mg/kg, more preferably from 10 to 30 mg/kg.

The compounds of the present invention are suitable for the treatment of food intake disorders, preferably bulimia, anorexia, cachexia, obesity, or type II diabetus mellitus (non-insuline dependent diabetes mellitus). It is particularly suitable in decreasing body weight. The compounds of the present invention are also suitable in the prophylaxis or treatment of appetency disorders, such as in reducing the desire for sweets.

The compounds of the present invention, pharmaceutically acceptable salts, solvates, and crystal forms thereof may therefore be used as a medicament. Said use as a medicament or method of treatment comprises the systemic administration to subjects in need thereof of an amount effective to combat food intake disorders, in particular to decrease body weight.

The present invention also relates to the use of the presents compounds in the manufacture of a medicament for the treatment or the prevention of food intake disorders, particularly for decreasing body weight.

The present invention furthermore relates to a method of decreasing body weight in a warm-blooded animal, said method comprising the administration of an effective amount of a compound of the present invention, pharmaceutically acceptable salts, solvates, or crystal forms thereof.

The following examples are intended to illustrate the present invention and not to limit it thereto.

### Examples

### Pharmacological Methods

### Example 1: In-vitro determination of affinity to human CB1-Receptors

Binding affinity for CB₁ receptor was evaluated according to a modification of the Govaerts SJ, Hermans E and Lambert DM. "Comparison of cannabinoid ligand affinities and efficacies in murine tissues and in transfected cells expressing human recombinant cannabinoid receptors". Eur. J. Pharm. Sci. 2004, 23 (3): 233-43 described method. Human CB₁ receptor was transfected into HEK-293 cells and a stable clone obtained and membranes prepared from this clone was used as the membrane source for radioligand binding studies. [3H](+)-CP55940 was used as radioligand at a final concentration of 0.45 nM. The incubation buffer was Tris-HCl 50 mM, MgCl2 5mM, EDTA 1mM, BSA 0.5%, pH 7.4 and the non-specific binding was determined with 10µM of HU210. Samples were incubated at 37ºC for 60 min before filtration. Radioactivity on filters was measured with Wallac Winspectral 1414 counter by liquid scintillation in Ecoscint H (National Diagnostics, U.K.).

### Example 2: In-vitro determination of functional activity to human CB₁-Receptors

The binding of [³⁵S]GTPγS was carried out using a modification of previously published methods Meschler, J.P., Kraichely, D.M., Wilken, G.M AND Howlet, A.C. "Inverse agonist properties of N-(piperidin-1-yl)-5-(4-chlorophenyl)-1-(2, 4-dichlorophenyl)-4-methyl-1H-pyrazole-3-carboxamide HCl (SR141716A) and 1-(2-chlorophenyl)-4-cyano-5-(4-methoxyphenyl)-1H-pyrazole-3-carboxyl ic acid phenylamide (CP-272871) for the CB(1) cannabinoid receptor." Biochem. Pharmacol. 2000, 60, 1315-1323 and Govaerts SJ, Hermans E and Lambert DM. "Comparison of cannabinoid ligand affinities and efficacies in murine tissues and in transfected cells expressing human recombinant cannabinoid receptors". Eur. J. Pharm. Sci. 2004, 23 (3): 233-43.

Human CB₁ membranes were prepared from stably transfected CHO cells. Incubation mixtures consisted of CHO-CB₁ membrane preparation at a final concentration of 15 µg of protein, compound (dose-response curve between 3 nM and 3µM, final well concentration), Win 55,212-2 (0.3 µM final well concentration) and binding buffer (50 mM HEPES, pH 7.4, 100 mM KC1, 5mM MgCl₂, 1 mM EDTA, 0.1 % wt/vol bovine serum albumin, 5 µM GDP, saponin 10 µg/ml). Subsequently [³⁵S]GTPγS (0.25 nM) was added, samples were incubated for 60 minutes at 30 ºC and finally filtered. Radioactivity on filters was measured with Wallac Microbeta Trilux. Data analysis was performed by using Graph Pad Prism version 3.03 for windows. The bottom plateau of the analyzed sigmoid curves is considered the Eₘᵢₙ (Minimal efficacy) value and values of Eₘᵢₙ < 0 represents inverse agonism of the compounds.

The in-vitro determination of affinity to human CB₁-receptors (Ki, in nM) for compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, was 27 ± 10.

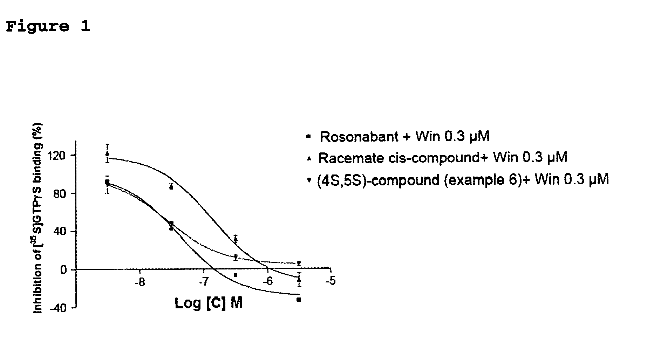

The in-vitro determination of functional activity to human CB₁-Receptors for compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide was: Emin = -1 ± 8, demonstrating that said compound acts as a neutral antagonist
pIC50 (potency) = 7.52 ± 0.1
See figure 1.
Said compound proved to be 99% completely selective for 170 targets.

### Example 3: Preparation of (E)-4-(4-Chlorophenyl)-3-methyl-2-oxo-but-3-enoic acid

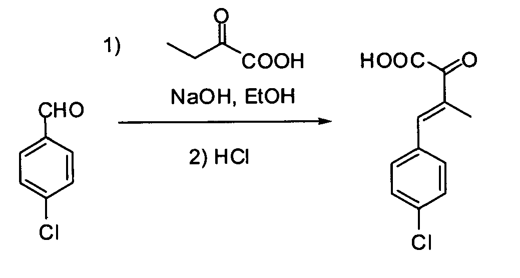

To a solution of aqueous 0.5 M NaOH (85.2 g, 2.13 mol 1.5 eq) in water (4.26 L), under N₂ at room temperature, 2-oxobutyric acid (159.7 g, 1.56 mol, 1.1 eq) was added in portions (60 mL of EtOH were used to wash the product container). The reaction was then left stirring for 5 min and a solution of 4-chlorobenzaldehyde (200.0 g, 1.42 mol, 1 eq) in abs. EtOH (710 mL) was then slowly added (approx. rate of addition: 2 h at 150 mL/h and 7h at 50 mL /h). The reaction was left to stir at 25 °C overnight. Water was added (800 mL) and the solution evaporated under reduced pressure to eliminate the excess of EtOH. The solution was then washed with toluene and evaporated (3 x 500 mL) to eliminate traces of this solvent. The aqueous solution was then cooled down in an ice bath and conc. HCl (240 mL) was slowly added under magnetically stirring. A white solid precipitated from the solution which was kept at 0 °C for another hour. The solid was filtered under vacuum through a sintered funnel (porosity 3) and dried at 40 °C under vacuum (287.8 g, 86% yield).

¹H NMR (400 MHz, CDCl₃): δ 2.17 (3H, s, CH₃), 7.44 (4H, ap d, J 3.28 Hz, ArH), 8.41 (1H, s, CH); ¹³C NMR (100 MHz, CDCl₃): δ 13.2 (CH₃), 129.2 (CH), 129.6 (C), 131.9 (CH), 133.4 (C), 136.4 (C), 147.6 (CH), 164.4 (CO), 187.1 (CO).

### Example 4: Preparation of racemate cis-5-(4-chlorophenyl)-1-(2,4-dichlorophenyl)-4-methyl-4,5-dihydro-1H-pyrazole-3-carboxylic acid

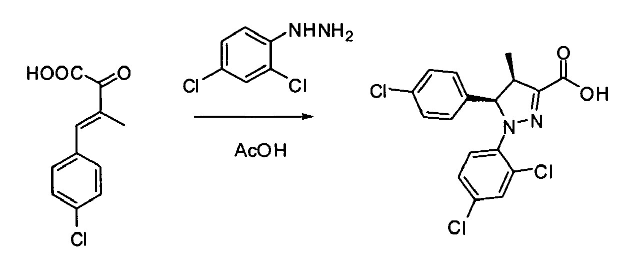

To a suspension of 2,4-dichlorophenylhydrazine hydrochloride (277.5 g, 1.27 mol, 1 eq) in acetic acid (2.0 L) at 80 °C, a solution of crude (E)-4-(4-chlorophenyl-3-methyl-2-oxo-3-butenoic acid (286.2 g, 1.27 mol) in glacial acetic acid (1.27 L) was slowly added and the reaction was maintained at 80 °C for 2 h. The reaction mixture was then allowed to cool down to 50 °C and concentrated under reduced pressure to approximately 2/3 of its initial volume. The solution was mechanically stirred at room temperature overnight and a yellow precipitated was formed. The solid was then filtered under vacuum through a sintered funnel (porosity 3) to obtain a mixture of racemates *cis* and *trans* 5-(4-chlorophenyl)-1-(2,4-dichlorophenyl)-4-methyl-4,5-dihydro-1*H*-pyrazol-3-carboxylic acid (430 g, 88.4 % yield) as a yellow solid, which was suspended in water (1.0 L), stirred for 30 min and filtered. This operation was repeated four times (until the pH of the filtered water was between 5 and 6). The solid was then left to dry under vacuum at 40 °C for 48 h (249 g, 51.2 % yield). The solid was suspended in toluene (1125 mL) and heated to 80 °C. The suspension was transformed into a solution, which was left at this temperature for 10 min and then at room temperature overnight. The following morning a yellow precipitated had formed. Filtration through a Buchner funnel under vacuum provided a whitish powdery solid of pure racemate *cis*-5-(4-chlorophenyl)-1-(2,4-dichlorophenyl)-4-methyl-4,5-dihydro-1*H*-pyrazol-3-carboxylic acid (189.7 g, 39 % yield, 76 % yield of crystallization).

IR (NaCl film, cm⁻¹ ) of the racemate *cis*-5-(4-chlorophenyl)-1-(2,4-dichlorophenyl)-4-methyl-4,5-dihydro-1H-pyrazol-3-carboxylic acid, measured with FT-IR Nexus by Thermo Nicolet: 2976, 1682, 1566, 1542, 1490, 1270, 1242, 1117.

¹H NMR (400 MHz, CHLOROFORM-*d*) δ ppm 0.96 (d, J=7.42 Hz, 3 H) 3.82 (td, J=11.72, 7.42 Hz, 1 H) 5.91 (d, J=11.72 Hz, 1 H) 7.04 (d, J=8.60 Hz, 2 H) 7.11 (dd, J=8.60, 2.34 Hz, 1 H) 7.21 - 7.30 (m, 4 H)

¹³C NMR (100 MHz, CDCl₃): δ 13.6 (CH₃), 43.5 (CH), 72.0 (CH), 124.9 (CH), 127.6 (CH), 129.1 (CH), 129.6 (CH), 130.9 (CH), 131.3 (C), 132.7 (C), 134.5 (C), 138.7 (C), 144.6 (C), 165.9 (CO).

### Example 5: Separation of (4R, 5R) and (4S, 5S)-5-(4-chlorophenyl)-1-(2,4-dichlorophenyl)-4-methyl-4,5-dihydro-1H-pyrazole-3-carboxylic acid from racemate cis-5-(4-chlorophenyl)-1-(2,4-dichlorophenyl)-4-methyl-4,5-dihydro-1H-pyrazole-3-carboxylic acid

### a) Separation by chiral HPLC.

Conditions for the isolation of each enantiomer of racemate *cis*-5-(4-chlorophenyl)-1-(2,4-dichlorophenyl)-4-methyl-4,5-dihydro-1H-pyrazole-3-carboxylic acid by preparative chiral HPLC were the following:
- Column: 250 x 20 mm CHIRALPAK^{®} AD-H 5 µm
- Mobile Phase: 80/20 CO₂ / Methanol
- Flow rate: 60 mL/min
- Detection: UV 230 nm
- Outlet Pressure: 150 bar
- Temperature: 25 º C

First eluting enantiomer, with a retention time of 5.56 min, was obtained with an enantiomeric excess higher than 99 %, and was identified as (4*S*, 5*S*)-5-(4-chlorophenyl)-1-(2,4-dichlorophenyl)-4-methyl-4,5-dihydro-1H-pyrazole-3-carboxylic acid (6.70 g, 47.8 % yield, [α]_{D}= +130.9 º). Second eluting enantiomer, with a retention time of 8.51 min, was also obtained with an enantiomeric excess higher than 99 %, and was identified as (4*R*, 5*R*)-5-(4-chlorophenyl)-1-(2,4-dichlorophenyl)-4-methyl-4,5-dihydro-1H-pyrazole-3-carboxylic acid (6.55 g, 46.8 % yield, [α]_{D}= -128.5 º).

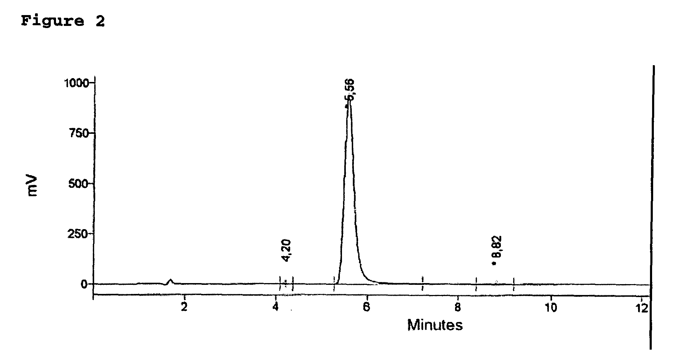

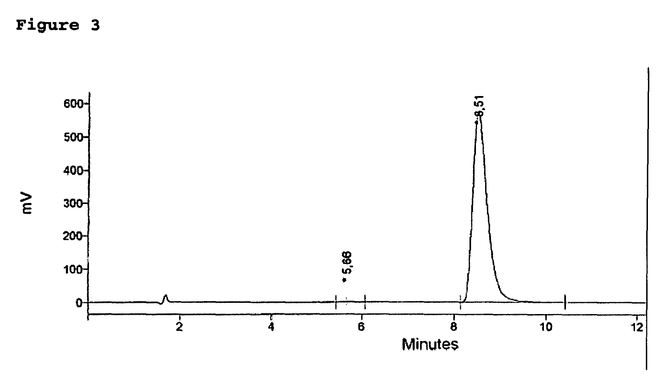

See figures 2 and 3.

### b) Diastereomeric resolution with chiral amines

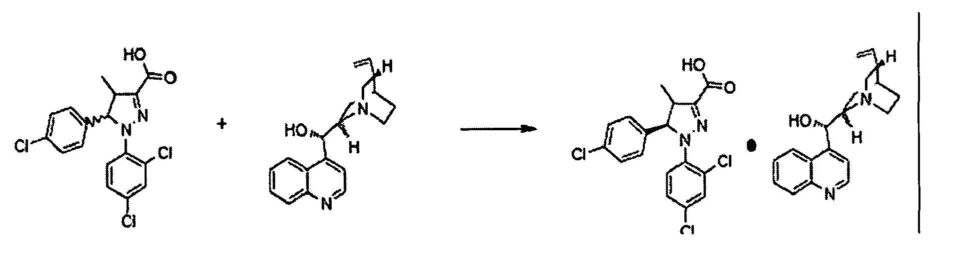

To a suspension of (+)-cinchonine in refluxing MeCN (2 mL), a solution of the racemate *cis*-5-(4-chlorophenyl)-1-(2,4-dichlorophenyl)-4-methyl-4,5-dihydro-1H-pyrazole-3-carboxylic acid (2.0 g, 5.2 mmol) in MeCN (2 ml) was added. To this mixture MeCN was added in small portions until all solids were dissolved. The solution was allowed to cool to r.t. overnight. White crystals were formed, collected by filtration and washed with MeCN. The crystals were dried under vacuum to obtain mainly (4*R*, 5*R*)-5-(4-chlorophenyl)-1-(2,4-dichlorophenyl)-4-methyl-4,5-dihydro-1H-pyrazole-3-carboxylic acid (1.7 g, 2.51 mmol, 48% (50% max), ee= 88.3 %). The acid was liberated by extraction of the white solid in toluene and 6 N HCl. The organic layer was dried over Na₂SO₄ and concentrated to give a light yellow foam (0.95 g, 2.47 mmol, 48 % yield, ee= 91.7%). To a solution of this foam in refluxing MeCN (2 ml), (+)-cinchonine (765 mg, 2.60 mmol) was added in a second crystallization process. To this mixture MeCN was added in small portions until all solids were dissolved. The solution was allowed to cool to r.t. overnight. White crystals were formed, collected by filtration and washed with MeCN. The crystals were dried under vacuum. The acid was liberated by extraction in toluene and 6 N HCl to give a white solid identified as pure (4*R*, 5*R*)-5-(4-chlorophenyl)-1-(2,4-dichlorophenyl)-4-methyl-4,5-dihydro-1H-pyrazole-3-carboxylic acid (860 mg, 42% yield, ee= 99.5%, [α]_{D}= -128.5 º). Recrystallization of mother liquors led to the (4*S*, 5*S*) enantiomer.

### Example 6: Preparation of (4S,5S)-5-(4-chlorophenyl)-1-(2,4-dichlorophenyl)-N-(cis-2,6-dimethylpiperidin-1-yl)-4-methyl-4,5-dihydro-1H-pyrazole-3-carboxamide

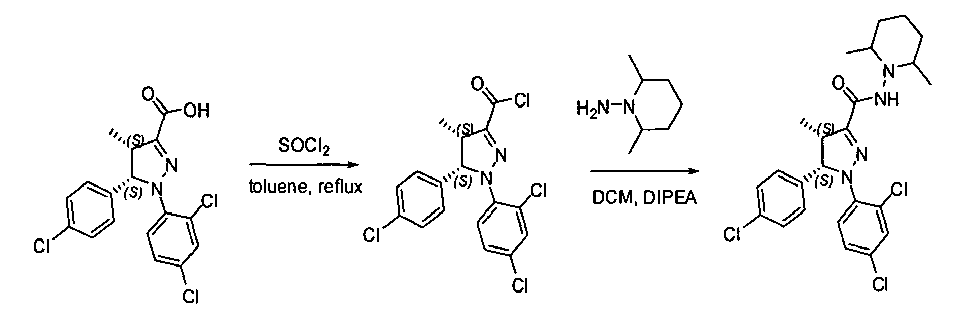

(4*S*,5*S*)-5-(4-chlorophenyl)-1-(2,4-dichlorophenyl)-4-methyl-4,5-dihydro-1H-pyrazole-3-carboxylic acid (20.0 g, 52.13 mmol) obtained according to the step above was dissolved in dry toluene (120 mL) and thionyl chloride (4.5 mL, 62.56 mmols) was added. The mixture was heated to 80 ºC for 2.5 hours. The solvent was removed under reduced pressure and the resulting crude residue was used without any further purification.

(4*S*,5*S*)-5-(4-chlorophenyl)-1-(2,4-dichlorophenyl)-4-methyl-4,5-dihydro-1H-pyrazole-3-carbonyl chloride was obtained as an oil.

IR (NaCl film, cm⁻¹) : 1732, 1700, 1533, 1478, 1212, 826.

Under nitrogen atmosphere *cis*-2,6-dimethylaminopiperidine (8.01 g, 62.56 mmoles) and diisopropylethylamine (DIPEA) (2.695 g, 208.5 mmol, 35.7 mL) were dissolved in methylene chloride (DCM) (100 mL). The resulting mixture was ice-cooled down to 0°C and a solution of (4*S*,5*S*)-5-(4-chlorophenyl)-1-(2,4-dichlorophenyl)-4-methyl-4,5-dihydro-1H-pyrazole-3-carbonyl chloride (20.96 g, 52.13 mmol), obtained in the former step, in methylene chloride (50 mL) was added dropwise. The resulting reaction mixture was stirred at room temperature (approximately 25 °C) overnight. Afterwards, the reaction mixture was washed with water, followed by a saturated aqueous solution of sodium bicarbonate, then again with water, dried over sodium sulfate, filtered and evaporated to dryness in a rotavapor. The resulting crude was purified by silicagel chromatography using a CombiFlash Companion apparatus, using a gradient of cyclohexane and ethyl acetate to obtain a white solid (18.3 g, 71 % yield).

¹H NMR (300 MHz, DMSO-d6) δ ppm 0.73 (d, *J*=7.18 Hz, 3 H) 0.95 (d, *J*=6.01 Hz, 6 H) 1.23 (br. s., 3 H) 1.63 (m, 3 H) 2.55 (br. s., 2 H) 3.83 (dd, *J*=11.13, 7.18 Hz, 1 H) 5.88 (d, *J*=11.13 Hz, 1 H) 7.12 (d, *J*=8.35 Hz, 2 H) 7.30 (m, 3 H) 7.45 (d, *J*=2.20 Hz, 1 H) 7.67 (d, *J*=8.79 Hz, 1 H) 8.64 (br. s., 1 H). MS (M+H)⁺: 535. [α]_{D}= +57.19 º.

Alternatively, a solution of 2 N HCl in diethyl ether were added over the solid dissolved in diethyl ether to form the hydrochloride, which was collected by filtration.

¹H NMR (300 MHz, DMSO-*d*₆) δ ppm 0.75 (d, *J*=7.32 Hz, 3 H) 1.05 (d, *J*=6.01 Hz, 6 H) 1.33 (m, 1 H) 1.47 (m, 2 H) 1.61 (d, *J*=9.96 Hz, 1 H) 1.72 (m, 2 H) 2.96 (br. s., 2 H) 3.87 (m, 1 H)) 5.92 (d, *J*=11.28 Hz, 1 H) 7.14 (d, *J*=8.35 Hz, 2 H) 7.32 (m, 3 H) 7.48 (d, *J*=2.20 Hz, 1 H) 7.65 (d, *J*=8.64 Hz, 1 H) 9.80 (br. s., 1 H). MS (M+H)⁺: 535.

## Claims

1. Compound (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, and pharmaceutically acceptable salts and solvates thereof.

2. The compound according to claim 1, wherein the salt is chloride.

3. The compound according to claim 2, wherein the salt is hydrochloride.

4. A process for the preparation of a compound according to any one of claims 1-3, said process comprising the step of:
a1) coupling a compound of formula (II) with *cis*-2,6-dimethylaminopiperidine in a suitable solvent, in the presence of a coupling agent, and optionally in the presence of an activating agent of the coupling agent, 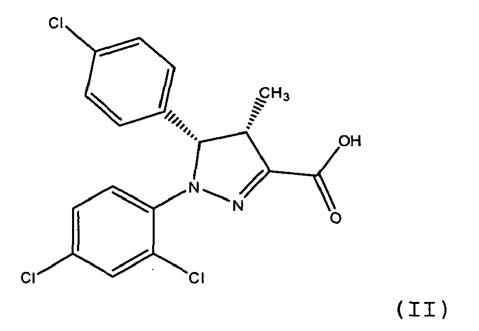 or
a2) converting a compound of formula (II) into a compound of formula (III) with an activating agent, and coupling said compound of formula (III) with *cis*-2,6-dimethylaminopiperidine in a suitable solvent, 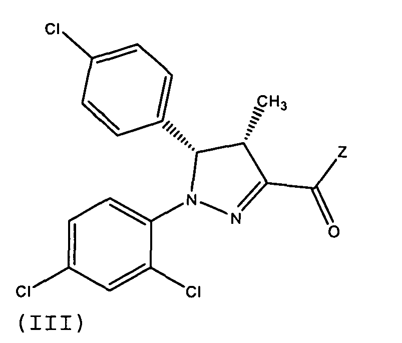 wherein
**Z** represents halo, -O-CO-R¹, or -O-CO-OR¹; and
**R¹** is C₁₋₄alkyl, aryl, or benzyl;
thereby obtaining compound according to any one of claims 1-3.

5. The process according to claim 4, wherein compound of formula (II) is obtained by chiral HPLC separation or by diastereomeric resolution with chiral amines of racemate *cis*-5-(4-chlorophenyl)-1-(2,4-dichlorophenyl)-4-methyl-4,5-dihydro-1H-pyrazole-3-carboxylic acid.

6. A compound of formula (II)

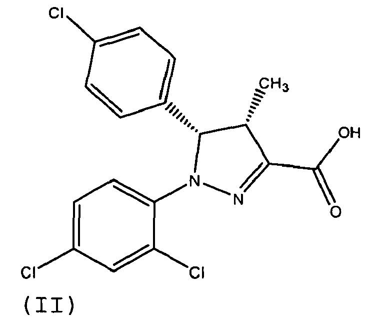

7. A compound of formula (III) 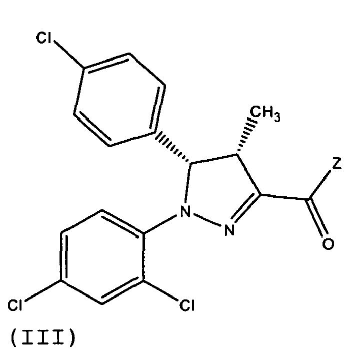 wherein
Z represents halo, -O-CO-R¹, or -O-CO-OR¹; and
**R¹** is C₁₋₄alkyl, aryl, or benzyl.

8. Compound (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide, and pharmaceutically acceptable salts and solvates thereof.

9. Use of a compound according to any one of claims 6-7 as an intermediate in the preparation of a compound as defined in any one of claims 1-3 and 8.

10. A pharmaceutical composition comprising a compound according to any one of claims 1-3 and 8, and a pharmaceutically acceptable carrier.

11. A compound according to any one of claims 1-3 and 8 for use as a medicament.

12. A compound according to any one of claims 1-3 and 8 for use as a medicament for decreasing body weight.

13. A method of decreasing body weight in a warm-blooded animal said method comprising the administration of an effective amount of a compound according to any of claims 1-3 and 8.
